Europäisches Patentamt

European Patent Office

Office européen des brevets

(19)

(11) Publication number: **0 218 468**
**A2**

(12) **EUROPEAN PATENT APPLICATION**

(21) Application number: **86307570.1**

(22) Date of filing: **01.10.86**

(51) Int. Cl.⁴: **C 12 N 15/00**, C 12 N 1/20
// (C12N1/20,
C12R1:125, 1:19, 1:385, 1:40)

(30) Priority: **01.10.85 JP 216209/85**

(43) Date of publication of application: **15.04.87
Bulletin 87/16**

(84) Designated Contracting States: **AT BE CH DE ES FR GB
GR IT LI LU NL SE**

(71) Applicant: **BANYU SEIYAKU KABUSHIKI KAISHA, 2-7-8,
Nihonbashi-honcho, Chuo-ku Tokyo (JP)**

(72) Inventor: **Mitsuhashi, Susumu, 1-35-11 Nishikubo,
Musashino-shi Tokyo (JP)**
Inventor: **Inoue, Matsuhisa, 3076-3, Tokisawa
Oaza-Fujimi-Mura, Seta-gun Gunma-ken (JP)**
Inventor: **Nagashima, Masao, 914-41, Kamiarai,
Tokorozawa-shi Saitama-ken (JP)**

(74) Representative: **Woods, Geoffrey Corlett et al, J.A.
KEMP & CO. 14 South Square Gray's Inn, London
WC1R 5EU (GB)**

(54) **Safe, versatile cloning vector.**

(57) A versatile recombinant DNA cloning vector has:

(a) two or more functionally different origins of replication selected independently from an origin of replication functionable in Bacillus subtilis, an origin of replication functionable in Escherichia coli and an origin of replication functionable in Pseudomonas aeruginosa, and

(b) one or more DNA sequence which is capable of providing resistance to at least one antibiotic when a susceptible host cell in which a said origin of replication is capable of functioning is transformed by the vector.

- 1 -

DESCRIPTION

SAFE, VERSATILE CLONING VECTOR

This invention relates to versatile cloning vectors and host cells transformed by them.

Many kinds of cloning vectors are known. However no versatile cloning vector which can function in different microorganisms such as Bacillus Subtilis (hereinafter referred to as Barillus), Escherichia coli (hereinafter referred to as E. coli) and Pseudomonas aeruginosa (hereinafter referred to as Pseudomonas) has generally been known. Only versatile cloning vectors which function in Streptomyces, Bacillus and E. coli (JP-A-58-134100) or in Corynebacterium and Brevibacterium (JP-A-58-105999) are known.

Important features of vectors in genetic engineering are described in "Recombinant Molecules; Impact on Science and Society", Miles International Symposium Ser. No. 10, Ed. by R.F. Beers, Raven Press, New York (1977).

In E. coli, plasmid vectors such as pBR322 have often been applied. The advantages of plasmid vectors, for example pBR322 can be illustrated as follows. (1) a higher copy numbers in cells which can easy be isolated ; (2) smaller molecular weight and limited site for restriction enzyme provide an easier cloning ability for DNA fragment without losing replication activity ; (3) easier selection on plasmid cloned bacterial cells by using selected marker, ampicillin-and tetracycline-resistance genes on a plasmid ; and (4) insertion of foreign DNA in cleavage sites of restriction enzyme in a plasmid, i.e. PstI site in ampicillin resistant gene and BamHI, HindIII and SaℓI sites in tetracycline resistant gene, transforming drug-resistance to drug-sensitive due to breakage of resistant gene ( insertional inactivation ), which provides easier selection of recombinant DNA holding bacteria by selecting plasmid holding cells with one drug resistant nature, followed selecting susceptibility to another drug. ( Bolivar. F. et al.: Gene, 2 : 95 ( 1977) ) . Many types of plasmid vector have prepared for E. coli. Vectros on the other industrially useful bacteria, for example amylase producing bacteria Bacillus, or waste decomposing strain Pseudomonas, have been developed. However practical example of application using these bacterial strains have not known, and it can be said that no advantageous plasmid vector as compared with that in E. coli has not found. Genes of E. coli is said that it could not expressed in Gram positive bacteria Bacillus in spite of the procaryote. Gene expression of palsmid is restricted and affected by background factors in transformed cells. For examples, transductant of drug resistant gene of E. coli jointing to plasmid of Bacillus and transforming in Bacillus can replicate its recombinant plasmid, but does not express drug resistant of E. coli. (Kreft, J., et al. Mol. Gen. Genet., 162 59 ( 1978,), Schottel, J. L., et al. ; J. Bacteriol.,146 360 ( 1981 ) ). A pBR322 and pACYC184 of E. coli vectors can not transformed in Pseudomonas. (" Plasmid of Medical, Environmental and Commercial Importance ", K.N. Timmis and A Puhler, eds. pp 411 - 422) .

Plasmid vector pBR322 or pCRI, which has replicant origin of pMBI or CoℓEI, is originally non-transferable plasmid, however they can be transferred together with coexisting transferable and another third non-transferable plasmid. (" Current Chemoth. and Immunotherap." Proc. 12th Internat. Cong. Chemoth. Vol. 1, p.6 ) . These may be dangerous to transfer drug resistant in a vector and unknown gene in a recombinant, and so these vectors are not preferable in a safety point of view.

Almost known plasmid vector developed in E. coli series is in its replication origin limited to coℓEI, pMBI, p15A and R6-5.When an insertion of plural plasmid vectors in a cell is required in order to increase productivity and to investigate gene relation, there may occur incompatibility, namely such like vectors can not coexist stably in a host cell and is eliminated.

A vector, RP-4, often used in Pseudmonas, in a large transferable plasmid of molecular weight 38Md, and is not a preferable vector. Furthermore, genetic information required for replication in RP-4 is separately located on a plasmid molecular and hence to prepare smaller vector plasmid is difficult. Only a plasmid RSF1010 and R $\ell$ b679 ( 5.5Md ) have been reported, however they are not preferable in view of insertional inactivation by using easily handled restriction enzyme.

Bacillus has advantages that

(a) is a production strain of antibiotics butyrocin and polymyxin.

(b) is a production starin of amilase and protease.

(c) is important industrial strain for exoenzymes. and

(d) is safe for foreign DNA recipient in a recombinant DNA technology due to non-parasitic in humans. However, development for vector is not progressed and no example in practice is found. One of reason is that drug resistant in E. coli can not express in Bacillus, and hence resistant marker of E. coli can not apply on Bacillus. Further effectiveness of transformation using competent cell is lower such as $10^2 \sim 10^3$ level per 1 $\mu$g of DNA as compared with that of E. coli series, and, whilst the use of the protoplast method improves the effectiveness of transformation, this method requires time and practice, so that the development of drug markers having preferable restriction site applicable to insertional inactivation might be delayed. Also, a shuttle-vector for Bacillus and E. coli has been reported [Abstract, Japan. Agr. Biol.Chem. Soc. Meeting 1980, p 408]. However the recombinant DNA technique on Bacillus expression of resistance against only one kind of drug can be observed and so no insertion-inactivation method is used.

An object of the present invention is to provide novel versatile cloning vectors which can function in Bacillus, Pseudomonas and E. coli and which can express one or more drug resistance in each microorganism.

We have investigated drug resistance gene markers which can be expressed in the Gram positive bacteria Bacillus and the Gram negative bacteria Pseudomonas and E. coli and selected the plasmid carrying strain. We have found that replication part of plasmid pMS71 from proteus mirabilis and that of plasmid pMS140-1 from Bacillus cereus jointed within the same plasmid can be replicated in Gram positive and negative bacteria, and further that the drug resistance from Gram positive bacteria linked with the same plasmid can also be expressed in each strain.

Accordingly, the present invention provides a versatile recombinant DNA cloning vector having;

(a)   two or more functionally different origins of replication selected independently from an origin of replication functionable in Bacillus subtilis, an origin of replication functionable in Escherichia coli and an origin of replication functionable in Pseudomonas aeruginosa, and

(b)   one or more DNA sequence which is capable of providing resistance to at least one antibiotic when a susceptible host cell in which a said origin of replication

- 5a -

is capable of functioning is transformed by the vector.

A novel plasmid of the present invention is prepared by considering the following advantages:

1. It can be replicated in a wide range of host cells.

Namely, effective selection of host cells that reveal good expression in one vector, and selection of genes and genetic product from various origin can be achieved.

2. Two kinds of drug resistant are expressed in any host cells. Selection of plasmid-borne cells and insertional-inactivation can be applied, and screening of recombinant plasmid-borne cells is easily performed.

3. Transformation can be made in E. coli system with good efficiency, without complicated operation or without less frequency of transformation as like in a Bacillus system, thereafter transformation to the desired host is possible.

4. Vectors should be large copying number easier isolation and smaller in size.

5. Vectors should be safety, namely not mobilizable any coexisting plasmids.

6. Restriction enzyme should be easily handled. Restriction enzyme recongnition site, especially for drug resistant site, should be one site in a vector for obtaining recombinant plasmid-borne cells.

7. It should be stably exist in host cells together with E. coli vector pCR1, pBR322, pACYC184, pSC101 or PBM9.( Never reveals incompatibility).

8. Drug resistant gene with high selective toxicity should be used as a selected marker.

9. Activity of selected marker is measured by an

0218468

- 7 -

amount of enzyme.

Plasmid vector of the present invention has been deposited Fermentation Research Institute, Japan and has benn assigned in a permanent deposit number as follows.

Escherichia coli ML4901-pMS163 ( A p$^r$ S a$^r$ ) FERM P-8288

Escherichia coli ML4901-pMS163-2( A p$^r$ K m$^r$ ) FERM P-8289

Escherichia coli ML4901-pMS163-5( A p$^r$ K m$^r$ ) FERM P-8290

Escherichia coli ML4901-pMS505-1( A p$^r$ S m$^r$ ) FERM P-8291

Escherichia coli ML4901-pMS504 ( A p$^r$ T C$^r$ ) FERM P-8292

Escherichia coli ML4901-pMS506 ( S m$^r$ T C$^r$ ) FERM P-8293

Advantage of the present invention is to provide a plasmid vector for genetic engineering process in host cells of genus Bacillus, genus Pseudomonas and geneus Escherichia . According to the advantage of the present invention, gene which can express in eucaryote and procaryote is cloned in vitro, thereafter it can be transformed in Escherichia coli , Pseudomonas aeruginosa. Pseudomonas putida, and Bacillus subtilis to select effectively desired transformed cells. More particularly, advantages of the present invention is that plasmid vector-borne cells can easily be selected by providing one or more drug resistance to host cells. It is therefore possible to select streptomycin sensitive cells by insertion of gene into EcoRI or HindIII site in streptomycin resistant gene, and further to select ampicillin sensitive bacteria by inserting gene into PstI or Bg$\ell$II site. By this way plasmid borne cells is selected with one antibiotic agent, therafter by checking sensitivity to another drug, recombinant plasmid-borned cells can be selected.

A vector of the present invention can be constructed by combining (1) a replication origin functioned in Bacillus, (2) a replication origin functioned in E. coli and Pseudomonas, (3) DNA segment which provides antibiotic resistance to E. coli and Pseudomonas, and (4) DNA segment which provides antibiotic resistance to Bacillus, E. coli and Pseudomonas. Combining (2) and (3) hereinabove provides a plasmid which can replicate in E. coli and Pseudomonas together with expressing antibiotic resistance in the both microorganism strains, can be obtained. Combination of a plasmid obtained by ligating (1) and (2) with the above DNA segment (4) provides a plasmid which can replicate in E. coli, Bacillus and Pseudomonas and can expresse antibitic resistance in any of E. coli, Bacillus and Pseudomonas.

DNA segment which can be replicated in Bacillus may be any plasmids originated from eucaryote and procaryote, and is preferably a plasmid of procaryote, especially Gram positive bacteria, selected from Staphylococcus, genus Streptococcus, genus Corynebacterium, genus Clostridium , genus Brevibacterium, genus Bacillus or Actinomycetes, and phage.

DNA segment which can be replicated in E. coli and Pseudomonas may be any plasmids originated from eucaryote and procaryote, and is preferably a palsmid of procaryote, especially Gram negative bacteria, selected from genus Escherichia, genus Pseudomonas, geneus Klebsiella, genus Salmonella, genus Alcaligenes, genus Flavobacterium, genus Enterobacter, genus Citrobacter, genus Haemophilis, genus Neisseria, genus Shigella, genus Acinetobacter, genus Campylobacter, genus Vibrio, genus Proteus, genus Serratia or Ercinia, and phage. Any plasmid of one replication origin which can replicate in both of E. coli and Pseudomonas, or any plasmid, either artificially or naturally ligated with segment from a replication origin in E. coli and that in Pseudomonas can be used. Plasmid, which carries antibiotic resistance markers and has selectivity in any of Bacillus, E. coli and Pseudomonas, from any of eucaryote and procarypte, can be used in the present invention. Preferable example of DNA segment carrying resistance markers is plasmid from procaryote, especially Gram positive bacteria, which is resistant to antibiotics such as $\beta$-lactam antibiotics, aminoglycosides, tetracyclines, chloramphenicol and macrolides, however any type of selection such as resistance to heavy metals and auxotrophic nature can be used.

A vector of the pressent invention which carries resistance to tetracycline, ampicillin, streptomycin, kanamycine or sulfa drugs, is preferable for checking the maintenance of the plasmid vector in host cells. Bacterial cells containing recombinant plasmids can easily be screened by incubating with medium which contains tetracycline, ampicillin, streptomycin, kanamycin or sufa drug. Furthermore, bacteria carrying recombinant DNA can stably be maintained in a medium containing suitable concentration of antibiotic.

Pseudomonas group of microorganisms is known to decompose various type of compound such as follows.

(a) organic halogen compound which is widely used as solvent, fire-extinguisher, insulator and pesticide, and is difficult to decompose microbiologically often with high toxic.

Refer to : " Assimilation of DL-2-chloropropionate " in J. Bacteriol., 150 , 522 ( 1982 ).

(b) Aromatic compound : diffucult to decompose

Refer to : " Assimilation of n-octane " in " proc. Japan. Agr. Chem. Soc., 1983 " .

(c) Biphenyl type compound in lignin :

Refer to : " Decomposition of 5,5-dehydrodibenylic acid " in " proc. Japan. Agr. Chem. Soc., 1983 ".

(d) A$_{zo}$-dye :

Refer to : " Assimilation of p-amino azobenzene " in Europ. J. Appl. Microbiol., 12 : 189(1981)".

(e) Methanol :

Refer to : " Molec. gen. GENET., 178 : 375 ( 1980 ) ".

The versatile cloning vector of the present invention can be cloned with a gene of _Pseudomonas_, which produces various kinds of decomposing enzyme on difficult-decomposed compounds, or a gene of _Bacillus_, which produces useful enzyme such as amylase and protease, and the said cloned plasmid can be expressed in the host cells such as any microorganisms of _E. coli_, _Bacillus_ and _Pesudomonas._ Namely decomposition of compound, which can not easily be decomposed, and production of useful substances can be achieved in desired microorganisms.

As explained hereinbefore, vector of the present invention has a number of advantages. Namely, numbers of copying of plasmid is high. Cloned vector carries only one restriction enzyme recognition site. Cloning of DNA segment can be acheived without loss of replication activity. Tetracyclin resistance, streptomycin resistance ampicillin resistance, kanamycin resistance or sulfa drug resistance in plasmid can be used for selection marker, thereby screening of plasmid-borne cells is quite easy. Sole restriction enzyme recongnition site in a plasmid is _XbaI_ , _Bg ℓ II_ and _PstI_ in ampicillin resistance gene, _XhoI_, _C ℓ aI_, _SmaI_ and _Hind III_ in kanamycin resistance gene, _PstI_ in sulfa drug resistant gene, or _EcoRI_ and _HindIII_ in streptomycin resistant gene. Because of existing such like easily applicable restriction enzyme recognition site in the cloned plasmid, cloning of DNA segment can be

- 12 -

achieved without hindering DNA replication activity, and selection of recombinant is performed by insertional inactivation method.

A vector of the present invention can be transformed in any microbial cells of Bacillus, E. coli and Pseudomonas which is sensitive to an antibiotic that is former in the vector as a resistant gene, and hence ampicillin resistance, kanamycin resistance, sulfa drug resistance and streptomycin resistance are expressed in both of E. coli and Pseudomonas, or ampicillin resistance, tetracycline resistance and streptomycin resistance are expressed in any bacterial cells of Bacillus, E. coli and Pseudomonas. Therefore, screening of the plasmid vector holding cells in any microorganism cells is easier, and selection of recombinant by insertional inactivation is also quite easy. Namely a vector of the present invention has advantage to select host cell for production of genetic engineering product.

In order to keep safety on gene manipulation, spread of recombinant gene and drug resistance in plasmid should be prevented. The plasmid vector of the present invention is not self-transferable and is not transfered even coexisting with transferable plasmid, or coexisting together three members of transferable plasmid, non-transferable plasmid and plasmid of the present invention (non-mobilizable) Hence comparing with plasmid pBR322 or pCRI, which is transfered at coexisting with the above explained three members, the vectors of the present invention can be said as vector of high safety.

Further, drug resistant marker, i.e. sulfa drug resistance, of the pressent invention is a conversion of heat-labile dihydropteroate synthetase, and is different from a conversion of heat-stable enzyme of sulf-drug resistance in prior known chromosome, and hence is quite safe.

Moreover, the plasmid vector of the present invention is quite useful in view of an incompatibility in the host cell E. coli. Conventional plasmid vector used in E. coli at present are originated from co ℓEl, pMBI, p15A or R6-5 in its replication origin, for example vector plasmid pCRI, pBR322, pACYC184 and pSC101, and the plasmid vector of the present invention can be coexisted stably in E. coli host cells, i.e. different in incompatibility group. Therefore, in order to increase genetic product, in the case that many kinds of plasmid in one host cell are required, ( for example, if the product is produced through multi steps of multiple enzymatic action, the same multiple numbers of plasmid vectors which coexist stably are required, ) the plasmid vector of the present invention is preferable. Also, analysis of gene interaction can be advantageously made.

To obtain the versatile plasmid vector of the present invention are cultured the plasmid supplying

- 14 -

bacteria such as E. coli and Bacillus in a medium. Supplier microorganism such as Escherichia coli ML4901, Bacillus subtilis CRK3000, Pseudomonas putida Tn1126 or Pseudomonas aeruginosa PAO2142 is cultured in a conventional medium containing carbon sources such as molasses, glucose, dextrin and glycerol, nitrogen sources such as soy bean powder, peptone and amino acid mixture, inorganic salt such as a salt of sodium, potassium, calcium, ammonium and magnesium or phosphate, chlor and sulfate, and trace essential element for microorganisms growth.

As illustrated hereinbefore, examples of microorganism strain used as plasmid host cell in a present invention are Eschrichia coli ML4901, Bacillus subtilis CRK3000, Pseudomonas aeruginosa PAO2142 and Pseudomonas putida Tn1126. Examples of medium and culture conditions are illustrated below, but are only for illustrative and can be modified in general that are included in the presesnt invention.

Harvesting of bacteria :

1. Escherichia coli, Pseudomonas aeruginosa and Pseudomonas putida are cultured in the followings.

(a) Liquid medium for growth of microorganisms and isolation of plasmid.

L-broth : Bacto-tryptone     10 g

Yeast extract      5 g

NaCℓ          5 g

Glucose       1 g

Dissolve in 1ℓ it. of deionized water, heated and adjust pH to 7.4 with sodium hydroxide after cooling, then sterilize at 120 ℃ for 20 min.

(b) Agar medium

Lactose-bromthymol blue ( Lac-BTB ) agar medium :

| | |
|---|---|
| Meat extract | 10 g |
| Peptone | 10 g |
| Nacl | 5 g |
| Lactose | 10 g |
| Agar | 15 g |
| Bromthymol blue | 0.8 g |

Dissolve in 1ℓ it. of deionized water, heating, adjust pH to 7.4 with NaOH after cooling, then sterilize at 120℃ for 20 min.

2. _Bacillus_ _subtilis_ is cultured in the followings.

(a) Liquid medium :

Antibiotic medium 3 ( Bacto-Pennasay medium ) (Difco) 17.5 g dissolved in 1ℓ it. of deionized water and sterilized at 120 ℃ for 15 min.

(b) Agar medium :

Heart infusion agar medium ( Nissui Co.) 25 g, dissolved in 1ℓ it. of deionized water with heating and sterilized at 120 ℃ for 20 min.

Small culture : in 10 mℓ both in test tube.

- 16 -

Large culture : in 200m ℓ both in Sakaguchi flask.

Culture temp. : 30 ～ 37 ℃.

Plasmid is isolated from the thus cultured cells by conventional method preferable according to the following procedure.

Isolation of plasmid :

( Cleared lysate method )

(1) Bacterial cells in L-both 200 m ℓ are shake cultured at 37℃ for overnight.

(2) Centrifuge at 6,000 r.p.m. at 4 ℃ for 15 min. to collect bacterial cells.

(3) Resuspend cells in a mixture ( 30 m ℓ) of 10mM Tris-1mM EDTA ( pH 8.0 ), transferred into 50 m ℓ centrifugal tube, centrifuge at 6000 r.p.m. for 10 min.

(4) Suspend completely the precipitate in cold 25 % sucrose solution ( 10 m ℓ) [sucrose ( 25 g ) is dissolved in 50 mM Tris-1 mM EDTA solution ( pH 8.0 )( 100 m ℓ) ] .

Ribonuclease ( RNase ) is added ( final concentrattion : 20 $\mu$g/m ℓ ) and stirred at maximum speed by Bortex-mixer for 2 min.

(5) Freshly prepared 1 % lysozyme solution ( 1m ℓ) is added and shake well, then ice-cold for 5 min.

(6) Add 0.5 M EDTA ( pH 8.0 )( 2.0m ℓ), shake well, allow to stand for 10 minutes in ice-cooling.

(7) Add cold triton lytic mixture ( 16m ℓ) , gently revolve test tube with twice turning upside down, mixed gently, ice-cold for 15 min.

(8) Centrifuge at 30,000 r.p.m. at 4℃ for 20 min.

Gently transfer the supernatant into sterilized measuring cylinder.

(9) Add polyethylene glucose ( # 6,000 ) up to 10 % ( w/v ) and mix well, thereafter add 5 M NaCℓ with 1/9 volume, then stored at 4℃ for overnight.

(10) Centrifuge at 5000 r.p.m. at 4 ℃ for 10 min.

Discard supernatant, add ×10 diluted SSC solution ( SSC: 0.15 M NaCℓ and 0.015 M sodium citrate ) ( 5 mℓ ) and gently dissolve DNA.

(11) Gently mix cesium chloride 5 g and DNA sample 5mℓ.

(12) After complete dissolving, add ethidium bromide ( 10mg/ mℓ) ( 0.1 mℓ ) and ×10 diluted SSC solution ( 0.1 mℓ).

Centrifuge at 37,000 ∼ 40,000 r.p.m. for 35 ∼ 40 hours at 20 ℃.

(13) Stop centrifugation without applying break, pick up gently centrifugal tube radiate ultraviolet light at a side of tube to observe fluorescence of DNA.

(14) Collect fluorescent band of ethidium bromide through injection needle inserted into the side of the tube. Be careful not to contaminate with other band.

(15) Add equal amount of cesium chloride saturated isopropanol to the DNA sample, shake gently to decolorize ethidium bromide.

Repeat the same operation for 3 - 4 times.

(16) Discard scarlet colored supernatant solution.

Transfer DNA sample into dialysis tube, then dialyze against ×10 diluted SSC solution.

(17) Store the thus prepared ccc DNA.


(1)  Isolation from E. coli :

Follow the above procedure . Refer in detail to  " Assay method for drug susceptibility " S. Mitsuhashi Ed. p. 42 ~ 48.

(2)  Isolation from Bacillus :

Follow the above procedure, but in (6) hereinabove " cooling for 10 minutes " is replaced by " heating at 37℃ for 30 minutes."

(3) Isolation from Pesudomonas :

Follow the above procedure, but in (5) hereinabove " 1 % lysozyme solution " is used, and " (6) 0.5 M EDTA " addition is not done.

(4) Isolation of plasmid from the above strains can be simplified according to the method described in " Nucleic Acids Research " vol. 1. No. 6 ( 1979 ) p. 1511 ~ 1523, which is summarized as follows.

Isolation by simple method :

(1) Culture overnight with suitable medium.

(2) Collect broth ( 1.0 ~ 1.5mℓ ) into 1.5 mℓ Eppendorf tube and centrifuge at 10,000 r.p.m. for one min. to collect cells. Discard supernatant.

(3) Add lysozyme solution ( hereinafter called as solution 1 ) 100 $\mu$ $\ell$, mix cells with Bortex mixer Ice-cool to stand for 30 min.

(4) Add alkaline SDS solution ( hereinafter called as solution 2 ) 200 $\mu$ $\ell$, mix by repeating to reverse upside down the Eppendorf tube, and allow to stand on ice for 5 min.

(5) Stir with mixing 3M sodium acetate solution ( pH 4.8 )( hereinafter called as solution 3 ) 150 $\mu$ $\ell$, and allow to stand on ice for 60 min.

(6) Centrifuge at 10,000 r.p.m. for 5 min., collect supernatant solution ( approx. 400 $\mu$ $\ell$ ).

Add twice vol. of 99.5 % ethanol. Stirred and allow to stand at $-20°C$ for 30 min.

(7) Centrifuge at 10,000 r.p.m. for 3 min. discard supernatant solution.

Add 100mM sodium acetate-50mM Tris- H C $\ell$ buffer ( pH 8.0 ) 100 $\mu$ $\ell$ in the precipitate to dissolve pallet.

Add twice vol. of 99.5 % ethanol, stir and allow to stand at $-20°C$ for 5 min.

(8) Centrifuge at 10,000 r.p.m. for 3 min.

Discard supernatant solution and lyophilize.

Add $\times 10$ diluted SSC solution 100 $\mu$ $\ell$ to prepare sample for transformation and agarose gel electrophoresis.

Transformation of recombinant DNA plasmid can be made by modified conventional means.

Transformation:

(1) E. coli

E. coli is cultured in L-both for overnight.

Cultured broth ( 0.1m ℓ ) is inoculated in fresh L-broth ( 10m ℓ ) and shake cultured at 37℃ for 3.5 hours. Collected cells are washed with 10mM NaCℓ -10mM Tris- H C ℓ buffer solution ( pH 8.0 )( 5 m ℓ ). Washed cells are suspended in 75mM CaC ℓ $_2$-10mM Tris- H C ℓ buffer solution ( pH 8.0 )( 5 m ℓ ) and allow to stand at room temperature for 20 minutes. Repeat the same operation and allow to stand in ice to obtain competent cells.

Cold competent cells ( 200 μ ℓ ) and plasmid solution ( 20 μ ℓ ) are mixed, ice-cooled for 20 min., warmed at 42℃ for 2 min. followed by adding L-broth ( 1 m ℓ ) then shake cultured at 37 ℃ for 90 minutes. After centrifugation at 3,000 r.p.m. for 10 min., cells are washed with BSG solution ( 1m ℓ ) , then resuspend in BSG solution ( 1 m ℓ ) . 0.1m ℓ thereof or its diluted suspension ( 0.1m ℓ ) is spread on a BTB agar medium containing kanamycin 12.5 μ g/m ℓ , and incubated at 37 ℃ for 24 hours subjecting to transformation. In details, refer to Mitsuhashi, S. Ed. " Assay methods for drug susceptivility " p. 38 - 41.

(2) Bacillus :

Bacterial cells are inoculated in Bacto-pennasay medium ( 5m ℓ ) added with glucose 0.5% for overnight.
Collected cells are suspended in C - 1 mediun ( 0.5 m ℓ ) ,
and 0.2 m ℓ thereof is inoculated in C - 2 medium
( 20m ℓ ) and harvested at 37 ℃ up to late logarithmic
phase on growth, which is transfer to C - 2 medium ( 80 m ℓ ) ,
then weakly cultured at 30℃ for 2 hours.  Competent
cells are collected, suspended in ice-cold C - 3 medium
( 10m ℓ ) , fractionated each 0.2 m ℓ and stored at -80℃.

The competent cells ( 0.2 m ℓ ) is mixed with
C - 1 medium ( 1.8m ℓ ) added with 20mM magnesium chloride.
The said cell mixture ( 450 μ ℓ ) is mixed with the plasmid
solution ( 50 μ ℓ ) , incubated at 37 ℃ for 30 minutes and
spread or agar plate medium containing drug to achieve
transformation.  If it requires incubation time for
expression, centrifugally collected cells are harvested
in Bacto-pennacy medium at 37 ℃ for 1.5 hour and spread
on a drug containing agar plates.  Refer in detail to
J. Bacteriol., 98 : 1239 - 1247 ( 1969 ).  Composition
of the above C - 1, C - 2 and C - 3 medium is as follows.

C - 1 medium : Dissolve ammonium sulfate 160mg.
Dispotassium hydrogen phosphate 480mg, sodium citrate
80mg in deinonized water 75 m ℓ and sterilized under pressure.
To the said solution is added sterilized 10% magnesium
sulfate solution 0.16m, 25% glucose 1.6 m ℓ , 5mg/m ℓ
histigine, adenine and methionine, 0.4m ℓ each respectively,
and 5mg/m ℓ leucine 0.16 m ℓ to prepare C - 1 medium.

C - 2 medium :

In C - 1 medium, histidine, adenine, methionine and leucine are replaced by 10% casamino acid 0.16m ℓ ( pH 7.0 ) and 5m g ∕ m ℓ histidine ( 1.6m ℓ) to prepare C - 2 medium.

C - 3 medium :

In C - 1 medium, histidine, adenine, methionine and leucine are replaced by glycerol a adding up to final concentration in 5%.

(3) <u>Pseudomonas</u> <u>aeruginosa</u> and <u>Pseudomonas</u> <u>putida</u> :

Bacterial cells grown in the middle logarithmic phase of growth in L-broth ( 10 m ℓ) is collected by centrifugation, washed with 0.1M magnesium chloride solution ( 5m ℓ) and collected. Cells resuspended in 0.1M magnesium chloride ( 5m ℓ) is allowed to stand in ice 20 minutes, then collected by centrifugation. Cell pellet suspended in 0.1M magneesium chloride ( 1 m ℓ) to obtain competent cells.

The competent cells ( 200 μ ℓ) and plasmid solution ( 50 μ ℓ) is mixed, allow to stand in ice for 60 minutes, thereafter warmed at 42 ℃ for 2 minutes, added L-broth ( 1 m ℓ) therein, and harvested at 37 ℃ for 90 minutes. Centrifugally collected cells is washed twice with BSG, added BSG ( 0.5 m ℓ) thereto, and spread on drug containing Lac-BTB agar medium, which is cultured at 37 ℃ to make transformation. Refer in details is

0218468

- 23 -

Antimicrobial Agent and Chemotherapy. Sep. 1982, p. 358 ∼ 363.

Checking transformation can be made by conventional means as illustrated in the followings.

Check points after transformation :

(1) Transformation is screened by resistant maker in a vector.

(2) Check desired drug resistant or not. In general, each test organism is spread on an agar plate medium containing drugs and observe its growth, then single colonies are selected twice on a plate containing drug.

(3) Isolate DNA by simple method, check an object molecular size on agarose-gel electrophorese and contaminants. An objectd DNA is re-transformed and confirm existence of the target gene maker on a plasmid. If necessary, check by cleaving with restriction enzyme.

(4) Isolate DNA by cleared lysate method for large scale preparation. Re-check by agarose-gel.

(5) For next step process, use DNA prepared in (4) above. Restriction enzyme and other enzymes used in molecular cloning can be available in commercial.

Isolated plasmid can be modified to a required plasmid for the present invention by generally recognized genetic engineering process. Preparation of the plasmid of the present invention is illustrated bellow.

Preparation of pMS500 :

- 24 -

A pMS140-1 is cleared at outer side of Tc$^r$ region by restriction enzyme EcoRl in single site. pMS71-17 is Ap$^r$ Sa$^r$ Both plasmids are completely digested by EcoRl to linear sequence, which is treated with T4 DNA ligase to prepare conjuagted molecule jointed togther with both DNA molecules at cohesive ends protruding ends of single chain. Recombinant circled DNA is once selected by drug resistance derived from pMS71-17. Transformant of E. coli ML4901 is isolated and the strain carrying Tc$^r$ is selected therefrom. Plasmid DNA is isolated from these Ap$^r$ Tc$^r$ strain by conventional method, and the recombinant plasmid in which both plasmids are ligated can be selected according to a result of agarose-gel electrophoresis.

A plasmid holding in the thus obtained transformation strain is a shuttle vector which can be replicated in Bacillus , E. coli and pseudomonas. Tc$^r$ maker derived from Gram positive bacteria can be express in Bacillus, E. coli and Pseudomonas, however Gram Ap$^r$ Sa$^r$ maker derived from Gram negative bacteria and Gram negative resistant maker Ap$^r$ Sa$^r$ does not express in Bacillus. As explanied hereinbefore, drug resistant gene of E. coli transformed in Bacillus is said not to express.

If two different drug resistances are expressed in the plasmid foreign plasmid DNA is cloned into another resistant DNA to transform resistance to sensitive, and

- 25 -

the transformant can be selected by using another drug resistance.

Preparation of pMS-500-1 :

pMS500 is digested with restriction KpnI and HindIII. By this treatment, Ap$^r$ which can not be expressed in Bacillus is removed and resulted smaller vecotr plasmid can be expressed in E. coli, Bacillus and Pesudomonas with carrying Tc$^r$ gene. The thus obtained vector plasmid is treated with SI nuclease to change cohesive end to blunt ended. Both ends are ligated by T$_4$ DNA ligase and transformed into E. coli, then selected Tc$^r$ colonies which is confirmed as Ap$^s$ . Plasmid is isolated from the Tc$^r$ transformant and check the molecular weight of the plasmid DNA by agarose-gel electrophoresis.

Preparation of pMS502 :

In order to insert secondary drug resistant in pMS500-1, which can express in E. coli, Bacillus and Pseudomonas, pMS500-1 is digested with restriction enzyme PstI at PstI region where there is a sole single part without relation to replication in each host cell, to prepare linear cohesive ended. Sm$^r$ in pMS501 can be replicated in E. coli and Bacillus, has a replication origin of E. coli vector pACYC177 and has sole cleavage site of PstI at outer side of Sm$^r$ gene. pMS501 is digested with restriction enzyme PstI and jointed with pMS501-1 by treatment with T$_4$ DNA ligase to preapre Tc$^r$ Sm$^r$

- 26 -

trnasformant pMS502. Plasmid pMS502 can be replicated in Bacillus, E. coli and Pseudomonas and provide Tc $^r$ Sm $^r$ in these strains.

Preparation of pMS502-1 :

HindlII and EcoRI site, which are useful for recombinant gene technology, are located within Sm$^r$ gene in pMS502. Plasmid pMS502 is digested with restriction enzyme AccI to delete regions between two AccI sites, and remaining sequence is jointed in each end by T$_4$ DNA ligase to obatin pMS502-1.

Preparation of pMS503 :

Two EcoRI sites are located in pMS502-1.

pMS502-1 is partially digested with EcoRI. A p $^r$ in plasmid pTTE11 ( Tc $^r$ Ap$^r$ ) is derived from Bacillus lickeniformis ( J. Bacteriol, 776 - 786 ( 1981 ) and can be expressed in E. coli and Bacillus. Plasmid pTTE11 is digested with EcoRI to prepare DNA fragment containing Ap$^r$ , which is jointed with pMS502-1 and transformed in E. coli to prepare Tc$^r$ Sm$^r$ Am$^r$ carrying plasmid vector pMS503.

Preparation of pMS503-1 :

Plasmid pMS503 has a region between two SacI sites in both side of EcoRI, which is not required for drug resistance and desired replication, and is digested with SacI to delete the fragment and ligate by T$_4$ ligase. Replicant's DNA is checked by agarose-gel electrophoresis to prepare plasmid pMS503-1.

pMS503-1 per se can be used plasmid vector, however EcoRI and HindIII sites are located in another region other than Sm$^r$ gene. Also PstI is located another region other than Ap$^r$ gene.

Preparation of pMS504 :

Restriction enzyme HaeII cleavage site is located in outer part of Sm$^r$ gene in pMS503-1. Plasmid pMS503-1 is digested with HaeII, ligated by T$_4$ DNA ligase and transformed in E. coli to obatin Ap$^r$ Tc$^r$ Sm$^s$ strain, in which PstI site located in outer side of Ap$^r$ so that PstI and Bg$\ell$II site in Ap$^r$ gene can be applied for insertional inactivation region. The other restriction enzyme site preferable for cloning in this plasmid is, for example, HindIII and EcoRI and SstI(SacI). Plasmid pMS504 can be replicated in E. coli, Pseudomonas and Bacillus together with expressing Ap$^r$ Tc$^r$.

Preparation of pMS506 :

Plasmid pMS503-1 that has SacI site located in one side of Ap$^r$ is digested with restriction enzyme SacI. Next partial digestion with EcoRI and treatment with SI nuclease are performed to prepare blant-ended plasmid, which is ligated with T$_4$ DNA ligase and transformed into E. coli to prepare Tc$^r$ Sm$^r$ Ap$^s$ strain. The thus obtained plasmid has lost HindIII and EcoRI sites outside of Sm$^r$ gene so that HindIII and EcoRI site in Sm$^r$ can be used for insertional inactivation region. Other preferable cloning region is for example singly located PstI and PvuI sites. Plasmid pMS506 can be replicated in E. coli, pseudomonasu and Bacillus together with expressing Tc $^r$ Sm $^r$ .

Preparation of Ap $^r$ Sm$^r$ /pMS505 :

Two restriction enzyme PvuII cleavage sites are located at both side of Tc$^r$ gene in pMS503-1, which is digested with PvuII and ligated with T$_4$ DNA ligase and selected Ap $^r$ Sm$^r$ Tc$^s$ . E. coli and HindIII sites in Sm$^r$ gene of the plasmid thus prepared can be used as insertional inactivation site, however PstI site is located other than PstI site in  Ap$^r$ gene.  So following treatment is necesary.

Preparation of Ap $^r$ Sm$^r$ /pMS505-1 :

Plasmid pMS505 is digested with PvuI,  partially digested with PstI, treated with nuclease BAL31 to remove bases of both ends for blunt-ended, thereafter ligated by T$_4$  DNA ligase and transformed in E. coli. After selecting Ap$^r$ Sm$^r$ strain, a plasmid DNA isolated from  Ap$^r$ Sm$^r$ cells is checked only one location of PstI site.

The thus prepared  plasmid pMS505-1 has an insertional inactivation region for Sm$^r$  gene such as HindIII and EcoRI sites, and that for Ap$^r$ gene such as PstI and Bg $\ell$ II sites.  The other restriction enzyme site located in this plasmid is SacI and Acc I.  The  Ap $^r$ Sm$^r$ strain can be replicated in E. coli and Pseudomonas to express  Ap $^r$ Sm$^r$ , however no replication is observed in Bacillus.

ADVANTAGE OF THE INVENTION :

The plasmid the thus prepared have specific useful features of plasmid as follows.

(1) Large copy numbers and easier selection .

(2) Selected markers as like tetracycline resistance, ampicillin resistance and streptomycin resistance or kanamycin resistance and sulfa resistance, in the plasmid can be used.

(3) In the plasmid, sole restriction enzyme cleavage sites, i.e. PstI and Bgℓ II and XbaI sites located in ampicillin resistat gene, EcoRI and HindIII sites located in streptomycin resistant gene, XbaI, ClaI, SmaI and HindIII located in kanamycin resistant gene, and PstI located in sulfa drug resistat gene, are located, and are advantageously used. Therefore, cloning of DNA segment can be performed without lossing DNA replication activity, moreover recombinant plasmid can be easily selected by insertional inactivation method.

(4) Plasmid can be transferred in any cells of Bacillus, E. coli and Pseudomonas which are sensitive to antibiotics provided as resistant gene. In any cells of Bacillus, E. coli and Pseudomonas, ampicillin resistance, tetracycline resistnce and streptomycin resistance, or that of E. coli and Pseudomonas, amplicillin resistance, kanamycin resistance sulfa drug resistance and streptomycin resistance are expressed, so that in any bacterial cells selection of

- 30 -

recombinant DNA can be selected by recombinant DNA technique and insertional method. Selection of host cell for purpose of production of substance by recombinant DNA technology is free of choice.

(5) In E. coli cells, the plasmid of the present invention does not mobilize with coexisting transferable plasmid or together with another non-transferable plasmid.

(6) The plasmid of the present invention can be stable with vector plasmid pCR1, pBR322, pACYC184 and pSC101 originated from Co $\ell$ E1, pMB1, p1SA and R6-5 in E. coli host cell. ( Different in incompatibility group ).

Following examples illustrate the present invention but are not construed as limiting.

In examples, buffer solutions for restriction enzyme and other genetic engineering techniques, condition of denaturing enzyme, conditions of washing and concentration of DNA solution, and abbreviation are illustrated as follows.

(1) Buffers for restriction enzyme :

Refer to " Molecular cloning ", Cold spring Harvor Laboratory, 1982, p. 104.

| Buffer | Na C $\ell$ | Tris·Cl(pH7.5) | Mg C $\ell_2$ | Dithiothreitol |
|--------|--------|----------------|---------|----------------|
| Low    | 0      | 10mM           | 10mM    | 1mM            |
| Medium | 50mM   | 10mM           | 10mM    | 1mM            |
| High   | 100mM  | 50mM           | 10mM    | 1mM            |

0218468

- 31 -

(2) Other buffers :

(a) BAL31-nuclease :

$12mM$ $CaCl_2$

$12mM$ $MgCl_2$

$0.6mM$ $NaCl$

$20mM$ Tris-HCl ( pH 8.0 )

(b) SI-nuclease :

$0.28M$ $NaCl$

$0.05M$ sodium acetate ( pH 4.6 )

$4.5mM$ $ZnSO_4$

(c) $T_4$ DNA ligase :

$10mM$ $MgCl_2$

$20mM$ dithiothreitol

$50mM$ Tris-HCl

$1mM$ ATP

$50$ $\mu M$ bovine serum albumin

Each buffer is prepared by adding inorganic salt in distilled water or directed buffer specialized for each restriction enzyme to set up fixed concentration and sterilized through milipore filter.

(d) TE :

$10mM$ Tris-HCl ( pH 8.0 )

$1mM$ EDTA ( pH 8.0 )

(3) Denaturation of enzymes in enzymatic reaction:

PstI and EcoRI : at 70 °C for 5 min.

Other enzymes : Equal volume of phenol is added to DNA solution, Vigorously shaken, centrifiged and transfer
DNA into Eppendorf tube. Add equal amount of ether to DNA
shake vigorously and discard upper ether layer. The same
are operations are repeated for 4 times to remove enzyme.

(4) Washing and concentration of DNA solution :

Add ethanol up to maximum concentration at 70%,
allow to stand at -20 ℃ for 60 minites , centrifuge at
10,000 r.p.m. for 5 minutes and discard upper layer.
Prior to ethanol addition, salt concentration in DNA solution
should be increased, generally to add 5M N a C ℓ  in 1/50
equivalent 3M sodium acetate in 1/10 equivalent.

(5) Abbreviations :

A p $^r$ : ampicillin resistance

A p $^s$ : ampicillin sensitive

T c $^r$ : tetracycline resistance

T c $^s$ : tetracycline sensitive

Sm $^r$ : streptomycin resistance

Sm $^s$ : streptomicin sensitive

Km $^r$ : kanamycin resistance

Km $^s$ : kanamycin sensitive

S a $^r$ : sulfa drug resistancce

S a $^s$ : sulfa drug sensitive

Example 1

Preparation of pMS71 for proteus  mirabilis  GN5404 :

Cultured both ( 1.5m ℓ ) of proteus  mirabilis
GN5404 at 37℃ for overnight in L-broth was introduced into Eppendorf tube and centrifuged at 10,000 r.p.m. for 1 minute. Collected cells were washed with TE buffer, centrifuged at 10,000 r.p.m. for 1 minute, then discard supernatant washing solution. Cells are suspended by Bortex mixer. Cold solution I ( 50mM glucose, 10mM EDTA, 25mM Tris-HCℓ buffer, pH 8.0 2mg/ mℓ lysozyme )(100 $\mu$ℓ) was added, stirred and allow to be kept in ice for 30 minutes. Solution II ( 0.2N NaOH, 1% lauryl sulfate ) ( 200$\mu$ℓ ) was added thereto mixed by 5 ~ 10 turns and kept in ice for 5 minutes. Cold solution III ( 3M sodium acetate, pH 4.8 ) ( 150$\mu$ℓ) was added, mixed and allowed to stand for 60 minutes. Supernatant solution ( approx. 400 $\mu$ℓ ) centrifuged at 10,000 r.p.m. for 5 minutes was transferred to another Eppendorf tube. Twice volume ( 800 $\mu$ℓ) of cold ethanol was added, mixed and allowed to stand at -20 ℃ for 30 minutes. After centrifigation at 10,000 r.p.m for 3 minutes, clear supernatant was discarded and added solution IV ( 0.1M sodium acetate 50mM, Tris-HCℓ buffer pH 8.0 ) ( 100$\mu$ℓ) to dissolve precipitate. Twice volume of cold ethanol was added, mixed, allowed to stand at -20 ℃ for 10 minutes, centrifiged at 10,000 r.p.m. for 3 minutes and collected the precipitate which was dried in desiccator. Precipitate is dissolved in TE buffer ( 100$\mu$ℓ) to prepare pMS71 for transformation.

- 34 -

Example 2

Transformation of pMS71 in E. coli ML4901 :

E. coli ML4901 was cultured in L-broth ( 10 m ℓ )
for overnight and shake cultured at 37℃.  On 3.5 hours
On 3.5 hours cultured when OD 560nm
is ovserved at 0.4, 5 m ℓ thereof was centrifuged at
3,000 r.p.m. for 10 minutes at room temperature, then
collected cells were washed with 10mM NaCℓ-10mM
Tris-HCℓ buffer ( pH 8.0, 5 m ℓ ) .  Cells collected
by centrifuge at 3,000 r.p.m. for 10 minutes were suspended
in 75mM CaCℓ$_2$-10mM Tris-HCℓ buffer ( pH 8.0, 5 m ℓ )
and allow to stand at room temperature for 20 minutes.
Cells again collected by centrifuge at 3,000 r.p.m. for
10 minutes were suspended in 75mM CaCℓ$_2$-10mM Tris-HCℓ
buffer ( pH 8.0, 5m ℓ ) and allowed to stand in ice for over
5 minutes to obtain compentent cells.

A plasmid solution ( 20 μ ℓ ) obtained in Example 1
which was stored in ice cold, in a sterilized small test
tube was added with cold competent cells ( 200 μ ℓ ) , mixed
gently and cooled in ice for 20 minutes, and trated with
heat for 2 minutes at 42℃ .  L-broth was added thereto
and shake cultured at 37℃ for 90 minutes.  After
centrifugation at 3000 r.p.m. for 10 minutes, bacterial cells
were washed with BSG  1 m ℓ ( NaCℓ 8.5 g, potassium
dihydrogen phosphate 0.3 g, disodium hydrogen phosphate 0.6 g
and gelatin 100 m g ∕ ℓ ), and resuspended in BSG ( 1 m ℓ )

and ten times diluted solution thereof ( 0.1m $\ell$ ) was spread on a Lac-BTB agar plate containing sulfonamide ( 100 $\mu$g/m $\ell$ ) and cultured at 37℃ for 24 hours. Transformant cells were twice cultured on a plate containing drugs and separated the plasmid according to the example 1, which was checked by agarose-gel electrophoresis.

Example 3

Identification of plasmid by agarose-gel electrophoresis :

0.8 % agarose 1600 ( Wako Pure. Chem. Co.) prepared with TEAS buffer ( 0.05M Tris-H C $\ell$, 2mM EDTA, 18mM N a C $\ell$ and 20mM sodium acetate, pH 8.0 ) was autocleaved at 120℃ for 10 minutes. On cooling at 65℃, agarose as take out, stirred well without forming, and poured gently into electrophoresis plate to stand. To DNA sample ( 10 $\mu$ $\ell$ ) was mixed with blue-juice ( 10 times concentrated TEAS buffer 2 m $\ell$, brom phenol blue 1.0 mg, sucrose 12 g, 0.5M EDTA 0.8 m $\ell$, distilled water up to 20m $\ell$ ) ( 1 $\mu$ $\ell$ ) and poured into gel. For 10 minutes electrophoresis at 60mA, blue juice was confirmed to move into gel, then it was electrophoresed at 25 mA for 12 hours. Gel was gently take-out from apparatus, strained with ethidium bromide ( 0.5 $\mu$g/m $\ell$ ) for 30 minutes and washed with dist. water, them gel was set-on the transiluminater UV-filter, and took photographed by polaroid type 57 film.

Example 4

Separation of DNA by cleared-lysate method :

pMS71/ML4901 obtained in Example 2 was shake cultured at 37 ℃ for overnight in L-broth 200 mℓ/500mℓ Sakaguchi flask.  Collected cells by 6,000 r.p.m. at 4℃ for 15 minutes was washed with TE. Cells collected was resuspended in a cold 25% sucrose solution ( 10 mℓ) ( 25% sucrose/50mM Tris- H C ℓ-1mM EDTA, pH 8.0 ).  Ribonuclease was added final concentration up to 20μg/mℓ, then transferred to 30mℓ volume centrifugal tube.  Mixiture was well mixed using Brotex mixer at highest revolution, then added 1% lysozyme ( 0.25M Tris-H C ℓ, pH 8.0 )(1mℓ), shaken well and cooled in ice for 5 minutes.  0.5M EDTA ( pH 8.0 )(2mℓ) was added and gently mixed and cooled in ice 10 minutes. Cold triton bacteriolytic solution ( 0.1% Triton x-100, 50mM Tris- H C ℓ, 62.5 mM EDTA, pH 8.0 ) ( 16mℓ) was added and turn to reverse upside down twice to gently mix, then cooled in ice for 15 minutes.  Solution was centrifuged at 30,000 r.p.m. for 20 minutes at 4 ℃, then supernatant was separated in 100 mℓ flask.  Polyethylene glycol-6000 was gently mixed for 10% ( w/v ), added 1/9 equivalent of 5M NaC ℓ to mix well and stored at 4 ℃, for overnight.  Mixture was centrifuged at 5,000 r.p.m. for 10 minutes at 4 ℃, discared supernatant and TE ( 5.3mℓ) was added to the residue to dissolve DNA. DNA sample ( 5.1mℓ) was gently mixed with cesium chloride ( 5g ).  After completely dissoloved, ethidium bromide

0218468

( 10m g / m ℓ ) ( 0.1 m ℓ ) was added , and mixed well, then centrifuged at 37,000 r.p.m. for 40 hours at 20 ℃. Over the transillminater plate, fluorescent band in test tube was collected ( 1 m ℓ ) by puncturing with the injection needle the side of the tube. Isopropanol saturated with cesium chloride was added with equal volume and gently shaken to decolourize ethidium bromide. This operation was repeated for three times to confirm complete decolorization on trans-illuminator UV filter, and transfered into dialysis tube then dialised against 0.1 SSC ( 15mM NaCℓ, 1.5mM sodium citrate ).

Example 5

Preparation of pMS71-18 :

Plasmid pMS71 ( 40 μ ℓ ) obtained by cleared method in Example 4 was added 10 times conc. buffer ( 500mM NaCℓ, 100mM Tris-HCℓ, pH 7.5, 100mM magnesium chloride and 10mM dithiothreitol )( 4 μ ℓ ) and restriction enzyme BamHI ( 2 μ ℓ, 3.2 units ) were further added, then reacted at 37 ℃ for overnight. pMS160-1 ( 40 μ ℓ) obtained by cleared lysate method was reacted with ten-times medium concentrated buffer ( 4 μ ℓ, hereinafter designates as × 10 conc. medium buffer ) adding BamHI ( 2 μ ℓ, 3.2 units) at 37 ℃ for overnight. Sample ( 10 μ ℓ) thereof was checked by agarose-gel electrophoresis to identify single cleavage of each plasmid. 3M sodium acetate ( 3 μ ℓ) and cold ethanol

( 80 $\mu$ $\ell$ ) were added to each plasmid solution and mixed well, allowed to stand at -80 ℃ for 30 minutes, then centrifuged at 10,000 r.p.m. for 5 minutes, discarded the supernatant solution and dried sediment in vacuo.

Each sample dissolved in sterilized distilled water ( 30 $\mu$ $\ell$ ) was reacted with ten times high concentrated buffer ( 3 $\mu$ $\ell$, hereinafter designates as ×10 high conc. buffer ) and EcoRI ( 2 $\mu$ $\ell$, 4.0 units ) at 37 ℃ for overnight. After checking the cleavage of plasmids by agarose-gel electrophoresis, 20 $\mu$ $\ell$ of each plasmid was mixed, added TE saturated phenol thereto and vigorously shaken. Mixture was centrifuged at 10,000 r.p.m. for 1 minute, and upper DNA layer transferred to another Eppendorf tube. Ether ( 40 $\mu$ $\ell$ ) were added thereto and vigorougly mixed well to transfer remained phenol to ether layer, then allow to stand and discarded the upper ether layer. After repeating ether treatment for 4 times, ethanol ( 80 $\mu$ $\ell$ ) was added DNA layer and allowed to stand at -80 ℃ for 30 minutes. Mixture was centrifuged at 10,000 r.p.m. for 5 minutes to discard supernatant solution and the sediment was dried in vacuo. Dried DNA was dissolved by adding $T_4$ DNA ligase buffer ( 30 $\mu$ $\ell$ ), and added $T_4$ DNA ligase ( 1 $\mu$ $\ell$ ) to react at 16℃ for overnight. Reaction mixture ( 20 $\mu$ $\ell$ ) was added competent cells ( 200 $\mu$ $\ell$ ) of ML4901 prepared by the same way as of Example 2 for transformation to obtain

transformant Sa$^r$ Ap$^r$ .

Agarose-gel electrophoresis and analysis using restriction enzyme indicated that the resulted transformant Sa$^r$ Ap$^r$ contains mutant lacking 0.1Md fragment including EcoRI site.  The former was designates as pMS71-17 ( 5.2Md ) and the latter as pMS71-18 ( 5.1Md).

Example 6

Preparation of pMS163 :

Mixture of pMS71-18 ( 30 $\mu$ $\ell$ ),  ×10  med. conc. buffer ( 3 $\mu$ $\ell$ ) and PvuII ( 2 $\mu$ $\ell$, 5.0 units ) was reacted at 37 ℃ for overnight.  Sample therof ( 10 $\mu$ $\ell$ ) was checked by agarose-gel electrophoresis for cleavage of plasmid.  Reaction mixture was subjected to phenol treatment and ether treatment as of in Example 5, thereafter added 3M sodium acetate ( 2 $\mu$ $\ell$ ) and cold ethanol ( 60 $\mu$ $\ell$ ) to mix well, then allowed to stand at -80 ℃ for 30 minutes. Mixture was centrifuges at 10,000 r.p.m. for 5 minutes, discarded supernatant solution, and the precipitate was dried in vacuo.  Precipitate dissolved in buffer  ( 40 $\mu$ $\ell$, for T$_4$ DNA ligase ) was ligated with T$_4$ DNA ligase, thereafter treated as same as in Example 5 to obtain transfomant Sa$^r$ Ap$^r$ .  Ten strains were treated as same in Example 1 to extract DNA analysed  by agarose-gel electrophoresis.  Plasmid of all 10 strains was same size of 3.9 Md which was designated as pMS163.

Example 7

Preparation of pMS163-1 :

Mixture of pMS163 ( 30 μℓ ), ×10 med. conc. buffer ( 3 μℓ ) and PstI ( 2 μℓ , 5.6 units ) were reacted at 30℃ for overnight. Also mixture of pNR1140 ( 30 μℓ ), ×10 med. conc. buffer ( 3μℓ ) and PstI ( 1μℓ , 2.8 units ) were reacted at 30℃ for overnight. Each DNA was analysed by agarose-gel electrophoresis to identify the cleavage site of PstI, thereafter both DNA solutions ( each 20 μℓ ) were mixed and treated with phenol and ether as same in Example 5. Also, ethanol prepitation and ligation by T₄ DNA ligase were taken as same in Example 5 to perform transformation. Transformation were spread on a Lac-BTB agar plate containing ampicillin 25μg/mℓ. Colonies appeared after incubation at 37℃ for overnight were checked resistances to kanamycin 25μg/mℓ and sulfonamide 50μg/mℓ to obtain a strain resistance to ampicillin and kanamycin and sensitive to sulfonamide. Plasmid DNA was isolated by cleared lysate method to identify molecular weight and cleavage by PstI, and identify the same as jointed plasmid of pMS163 and PstI fragment conatining kanamycin resistance in pNR1140.

Example 8

Preparation of pMS163-2 :

Plasmid pMS163-1 ( 50 μℓ ), ×10 high. conc. buffer ( 5 μℓ ) and SaℓI ( 1.5 μℓ, 1.5 unit ) were mixed and reacted at 37 ℃ of overnight. A 10μℓ thereof was

electrophoresis on agarose-gel to identify cleavage of plasmid, thereafter reaction mixture was mixed with cold ethanol ( 100 $\mu\ell$ ) and allowed to stand at -80 ℃ for 30 minutes, then centrifuged at 10,000 r.p.m. for 5 minutes to collect precipitate which was dried in vacuo. Nuclease SI ( 0.5 $\mu\ell$, 250 units ) was added to the precipitate dissolved in buffer ( 50 $\mu\ell$ )for SI nuclease and reacted at 30 ℃ for 30 minutes. TE saturated phenol ( 50 $\mu\ell$ ) was added therein, vigorously stirred, centrifuged at 10,000 r.p.m. for 1 minute and transferred the upper aqueous layer to another Eppendorf tube. Water saturated ether ( 50 $\mu\ell$ ) was added, stirred, allowed to stand and discarded the upper ether layer. The ether treatment was repeated for 4 times. Cold ethanol was added therto and again allowed to stand at -80℃ for 30 minutes, then centrifuged at 10,000 r.p.m. for 5 minutes, collected the precipitate and dried in vacuo. The precipitate was dissolved in sterilized distilled water ( 40 $\mu\ell$ ), added with ×10 low conc. buffer ( 4 $\mu\ell$ ) and Hpal ( 1 $\mu\ell$, 0.5 unit ), and the mixture was reacted at 37 ℃ for 30 minutes. Treatment by phenol, ether and ethanol precipitation were performed and dired in vacuo, then ligase treatment and transformation were taken as of Eaxample 5. Colonies grown on Lac-BTB agar containing ampicillin ( 25 $\mu$g/m$\ell$ ) was confirmed showing

kanamycin resistance. DNA of 50 strains thereof was analysed by agarose-gel electrophoresis to yield 15 strains having smaller size plasmid as compared with pMS163-1.

Example 9

Preparation of pMS163-4 :

Plasmid DNA of pMS163-1 ( 20 $\mu\ell$ ), ×10 low conc. buffer ( 2 $\mu\ell$ ) and HaeII ( 0.5 $\mu\ell$, 0.2 unit ) were mixed and reacted at 37 °C for 30 minutes. After treatment with phenol and ethanol, 3M sodium acetate ( 2 $\mu\ell$ ) was added. Further after ethanol precipitation, ligase reaction and transformation were performed as of in Example 5 to select ampicillin and kanamycin resistant strains. Plasmid 50 strains thereof were analysed by agarose-gel electrophoresis to yield smaller molecular weight plasmid.

Example 10

Preparation of pMS163-5 :

Plasimid pMS163-4 ( 50 $\mu\ell$ ), ×10 med. conc. buffer ( 5 $\mu\ell$ ) and PstI ( 2 $\mu\ell$, 5.6 units ) were mixed reacted at 30 °C for overnight, then checked the cleavage by agarose-gel electrophoresis. HindIII ( 1 $\mu\ell$, 1.2 unit ) was added therein, reacted at 37 °C for 30 minutes, and subjected to phenol and ether treatment. Further 3M sodium acetate( 4 $\mu\ell$ ) was added therto and treated with ethanol, then digested with SI nuclease as of in Example 8, and subjected to treatments by phenol, ether and ethanol precipitation. Precipitate thus obtained dissolved in buffer for $T_4$ DNA was reacted with ligase. Again treatments by phenol and ether and ethanol precipitation were taken. The thus obtained precipitation was dissolved in sterilized distilled water ( $40 \mu \ell$ ), mixed with $\times 10$ med. conc. buffer ( $4 \mu \ell$ ) and PstI ( $2 \mu \ell$ ) and reacted at 37 ℃ for 2 hours. Reaction mixture was transformed to competent cell of ML4901 strain and spread on Lac-BTB agar medium containing kanamycine $25 \mu g/m \ell$. Three strains of Km $^r$ Ap$^r$ were obtained and 2 strains thereof was fit the purpose.

Example 11

Preparation of pMS500 from pMS71-17 and pMS140-1 :

A $\times 10$ high conc. buffer ( $5 \mu \ell$, for restriction enzyme ) and EcoRI ( $0.5 \mu \ell$, 2.5 units ) were added to pMS140-1 ( $50 \mu \ell$, 1 $\mu g$ DNA ) and reacted at 37 ℃ for overnight. A pMS71-17 ( $50 \mu \ell$ ) was treated with EcoRI. Both reaction mixtures were combined and treated 70℃ for 5 minutes to stop the reaction, then 99.5% ethanol ( $220 \mu \ell$ ) was added thereto, mixed and allowed to stand at 20 ℃ for 60 minutes. Mixture was centrifuged at 10,000 r.p.m. for 5 minutes, discarded the supernatant solution, and dried in vacuo. A buffer for $T_4$-DNA ligase ( $50 \mu \ell$ ) and $T_4$-DNA ligase ( $0.2 \mu \ell$, 1 unit) were added therein and reacted at 16 ℃ for overnight. E. coli competent cell suspension ( $200 \mu \ell$ ) was added thereto,

reacted in ice for 20 minutes and treated at 42 ℃ for 2 minutes. L-broth ( 1 m ℓ ) was added, shake cultured at 37℃ for 90 minutes, centrifuged at 3,000 r.p.m. for 10 minutes. Collected cells were washed twice with BSG ( 1 m ℓ ) and spread on Lac-BTB agar medium containing ampicillin 25 μg/m ℓ . After incubating at 37℃ for overnight, grown colonies were spread on Lac-BTB medium containing tetracyclne 12.5 μg/m ℓ and ampicillin 25μg/m ℓ by using sterilized sticks, and colonies grown on agar plates were treated twice on the same drug concentration agar medium to obtain Ap $^r$ Tc $^r$ strain.

Example 12

Preparation of pMS500-1 from pMS500 :

Plasmid was obtained from strain carrying plasmid pMS500 by the previously described method. Prepared plasmid ( 20μ ℓ ), ×10 low conc. buffer ( 2 μ ℓ ) and KpnI ( 0.5μ ℓ, 4.5 units ) were mixed and reacted at 30 ℃ for overnight. 5M NaC ℓ ( 0.25 μ ℓ) and HindIII ( 0.5 μ ℓ, 6.0 units ) were added thereto and reacted at 37℃ for 5 hours. Further 3M sodium acetate ( 2μ ℓ) and 99.5% ethanol ( 50μ ℓ) were added and mixed, then allowed to stand at -20 ℃ for 60 minutes. Reaction mixture was centrifuged at 10,000 r.p.m. for 5 minutes, discarded the supernatant solution and dried in vacuo. SI nuclease ( 0.5 μ ℓ, 29 units ) was added to precipitate suspended with buffer ( 50μ ℓ, for SI nuclease ), and

reacted at 30 ℃ for 30 minutes, thereafter TE saturared phenol ( 50 μℓ ) was added. stirred vigorously by Bortex mixer ( approx. 5 sec.), then centrifuged at 10,000 r.p.m. for 1 minute and collected upper aqueous layer. Water saturated ether ( 50 μℓ) was added thereto, stirred ( approx. 2 sec.), Allowed to stand 1 minute, upper ether layer was discurded. The ether treatment was repeated for 4 times, and to the resulted aqueous layer ( approx. 50 μℓ) was added 99.5% ethanol ( 120 μℓ ) and allowed to stand at -20℃. After 1 hour solution was centrifuged at 10,000 r.p.m. for 5 minutes. Precipitate was washed with 70% ethanol ( 100 μℓ ) and again centrifuged, discarded the supernatant solution, dried in vacuo, suspended in a buffer for T$_4$ ligase, added T$_4$ DNA ligase ( 0.2 μℓ, 10 units ) and reacted at 16 ℃. for overnight. Transformation into E. coli performed as of in Example 11, and the transfrormant was spread on Lac-BTB medium containing tetracycling 12.5 μg/mℓ, then incubated at 37℃ for overnight. Colonies grown on the said medium and not grown at ampicillin 25 μg/mℓ were selected to obatin Tc$^r$ Ap$^r$ strain.

Example 13

Preparation of pMS502 from pMS500-1 and pMS501 :

A ×10   med. con. buffer ( 2 μℓ) and PstI ( 1μℓ, 5 units ) were added to pMS500-1 ( 20 μℓ) and reacted at 30℃. Further  ×10   med. con. buffer ( 5 μℓ) and PstI

( 1 $\mu\ell$, 5 units ) were added to pMS501 ( 50 $\mu\ell$ ) and reacted at 30 ℃ for overnight. Both reaction mixtures were combined. trated at 70 ℃ for 5 minutes to denature the enzyme, then the often operations were treated as same as of in Example 11, for example ethanol precipitaion, T₄ DNA ligase treatment and transformant into E. coli.

The thus obtained strains were selected by Lac-BTB medium containing tetracycline 12.5 $\mu$g/m$\ell$, and colonies grown on this medium were spread on Lac-BTB medium containing streptomycin 6.25 $\mu$g/m$\ell$, thereby selected the strain resistance to tetracycline 12.5 $\mu$g/m$\ell$, and streptomycin 6.25 $\mu$g/m$\ell$ to obtain Tc$^r$ Sm$^r$ strain.

Example 14

Preparation of pMS502-1 from pMS502 :

A 10× med. conc. ( 2 $\mu$$\ell$ ) and AccI ( 2 $\mu$$\ell$, 1.4 unit ) were added to pMS502 ( 20 $\mu$$\ell$ ) and reacted at 37 ℃ for overnight. Subsequnt operations were proceeded as same as of in Example 12, i.e. phenol treatment, ether treatment ( 5 times ) ethanol precipitation. T$_4$ DNA ligase treatment and transformation into E. coli. Resulted strains were selected by resistances to tetracycline 12.5 $\mu$g/m$\ell$. Plasmid isolated therefrom was checked by agarose-gel electrophoresis and selected the plasmid of smaller than plasmid pMS502 to obtain pMS502-1.

Example 15

Preparation of pMS503 from pMS502-1 and pTTE11 :

A ×10 high conc. buffer ( 2$\mu$$\ell$ ) and EcoRI ( 0.5$\mu$$\ell$, 0.25 unit ) were added to pTTE11 ( 20 $\mu$$\ell$ ) and reacted at 37℃ for overnight. A ×10 high conc. buffer ( 2$\mu$$\ell$ ) and EcoRI ( 1 $\mu$$\ell$, 0.5 unit ) were added to pMS502-1 ( 20 $\mu$$\ell$ ) and reacted at 37 ℃ for 10 minutes. Both plasmid solutions were combined, treated at 70°C for 5 minutes, and subsequent operations were treated as same as of in Example 11 to trasform into E. coli, colonies were selected at first grown on Lac-BTB medium containing streptomycin 3.13 μg/mℓ and at the secondary resistances to ampicillin 25 μg/mℓ and tetracycline 12.5 μg/mℓ to obtain Tc$^r$ Sm$^r$ Ap$^r$ strain. plasmid was checked by agarose-gel electrophoresis that original pTTE11 was not contaminated and size thereof as largerr than pMS502-1.

Example 16

Preparation of pMS503-1 from pMS503 :

A ×10 low conc. buffer ( 2 μℓ ) and SacI ( 0.5 μℓ, 1.25 unit ) were added to pMS503 ( 20 μℓ) and reacted at 37°C for overnight. Phenol ( 20μℓ) and ether ( 20 μℓ, 4 times ) treatments, ethanol concentration, reaction by T$_4$ DNA ligase and trasformation into E. coli were performed as same as of in Example 5. Colonies selected by streptomycin 3.13 μg/mℓ were confirmed to grown on tetracycline 12.5 μg/mℓ and ampicillin 25 μg/mℓ. Plasmid isolated from the colonies was identified by agarose-gel electrophoresis to show smaller plasmid.

Example 17

Preparation of pMS504 from pMS503-1 :

A ×10 low conc. buffer ( 2 μℓ ) and HaeII ( 0.5μℓ,

- 49 -

4 units ) were added to pMS503-1 ( 20 $\mu\ell$ ) and reacted at 37℃ for overnight.  Subsequent was proceeded as same as of in Example 16 and colonies were selected by ampicillin 25 $\mu$ g / m $\ell$ , and grown colonies were further selected growth on tetracycline 12.5 $\mu$ g / m $\ell$ , and no-growth on streptomycin 3.13 $\mu$ g / m $\ell$ to obtain Ap $^r$ Tc$^r$ Sm$^r$ strain.  Plasmid thereof was identified by agarose-gel electrophoresis as same in Example 16.

Example 18

Preparation of pMS506 from pMS503-1 :

A ×10  low conc. buffer ( 5 $\mu\ell$ ) and SacI ( 1.5 $\mu\ell$ , 2.5 units ) were added and reacted at 37℃ for overnight. A ×10  high conc. buffer ( 6 $\mu\ell$ ) was added thereto , and further added EcoRI ( 1 $\mu\ell$ , 0.5 unit ) then reacted at 37 ℃ for 15 minutes and thereafter treated at 70℃ for 5 minutes to stop the reaction.  99.5% ethanol ( 140 $\mu\ell$ ) was added thereto and allowed to stand at -20 ℃ for 60 minutes.  Further treatments by SI nuclease, phenol, ether ( 4 times ) and ethanol, and T$_4$ DNA ligase treatment were taken as same as of in Example 12 and transformation into E. coli.  Colonies grown on Lac-BTB medium containing streptomycin 3.13 $\mu$ g / m $\ell$ were selected, and checked this strain resistance to tetracycline to obtain Sm $^r$ Tc$^r$ Ap$^s$ strain.  Plasmid isolated according to a method described in Example 1 was identified by agarose-gel electrophoresis.

Example 19

Preparation of pMS505 from pMS503-1 :

A ×10 med. conc. buffer ( 2 μ ℓ ) and Pvull ( 0.5 μ ℓ , 0.5 unit ) were added to pMS503-1 ( 20 μ ℓ ) and reacted at 37 ℃ for 30 minutes. Phenol treatment, ether treatment, ethanol precipitation and T₄ DNA ligase treatment were perform as same as of in Example 12 and transformation into E. coli. Colonies grown on Lac-BTB medium containing ampicillin 25 μ g / m ℓ were selected and further identified as that grown on streptomycin 6.25 μ g / m ℓ and not growth on tetracycline 12.5 μ g / m ℓ to obtain Ap$^r$ Sm$^r$ Tc$^s$ strain.

Example 20

Preparation of pMS5051 from pMS505 :

A ×10 high conc. buffer ( 3 μ ℓ ) and Pvull ( 1 μ ℓ , 4 units ) were added to pMS505 ( 30 μ ℓ ) and reacted at 37 ℃ for overnight. 99.5% ethanol ( 70 μ ℓ ) was added thereto and allowed to stand at -20 ℃ for 60 minutes. Reaction mixture was centrifuged at 10,000 r.p.m. for 5 minutes, discarded the supernatant solution and dried in vacuo. Sterilized water ( 30 μ ℓ ), ×10 med. conc. buffer ( 3 μ ℓ ) and Pvull ( 1 μ ℓ , 0.5 unit ) were added to thereto and reacted at 30 ℃ for 30 minutes. Reaction mixture was treated at 70 ℃ for 5 minutes, added 3M sodium acetate ( 3 μ ℓ ) therein, then added 99.5% ethanol 70 μ ℓ , and the mixture thereof was allowed to stand at -20 ℃ for

60 minutes. Reaction mixture was centrifuged at 10,000 r.p.m. for 5 minutes, discarded the supernatant and dried the precipitate in vacuo. Precipitate was suspended in buffer for BAL31 ( 50μℓ ), BAL31 ( 0.5 μℓ, 1.25 unit ) was added thereto, and reacted at 30℃ for 10 minutes. TE saturated phenol ( 50 μℓ) was added and vigorously stirred by Bortex mixer, then centrifuged at 10,000 r.p.m. for 1 minute, and collected upper aqueous layer. Collected aqueous layer was treated with ether ( 5 times ), ethanol and T₄ DNA ligase as same as of in Example 12. Transformation into E. coli was performed as same as of in Example 11 and colonies were selected to grow on Lac-BTB medium containing ampicillin 25 μg/mℓ. Grown colonies were confirmed to streptomycin 6.25μg/mℓ resistance to obtain Ap$^r$ Sm$^r$ strain.

Plasmid isolated from Ap$^r$ Sm$^r$ strain according to a method described in Example 1 was identified that smaller than pMS505. Further PstI digestion indicated single cleavage site.

Example 21

Transformation of vector plasmid into Bacillus and Pseudomonas :

Plasamid vector ( 25μℓ ) obtained according to a description in " Method for assaying drug sensitivity " Ed, Mitsuhashi, p, 42 - 48, was used for transformation into Bacillus subtilis, Pseudomonas aeruginosa and Pseudomonas putida.

(A) Bacillus subtilus ( CRK 3000 ) :

Plasmid pMS504 or 506 ( 25 μ ℓ) obtained by the previous examples was mixed with competent cells of Bacillus subtilis ( CRK 3000 )( 200 μ ℓ) and shaked at 37 ℃ for 30 minutes. Mixture was spread on heart infusion agar plate containing tetracycline 6.25 μg/m ℓ and incubated at 37 ℃ for overnight, then grown colonies were cultured ( pure culture ) on tetracycline 1.25 μg/m ℓ containing medium. This strain was inoculated in assay medium for sensitivity test broth( Nissui Co ) and cultured at 37℃ for 18 hours. Cultured broth diluted with BSG to $10^5 \sim 10^6$ cells/ m ℓ, was inoculated 5 μ ℓ thereof on agar plate containing each kind of drugs with various concentration by using micro-planter. The plates were incubated at 37 ℃ for 18 hours and checked the growth of colonies. Result is shown in Table 1.

Table 1

MIC ( μg/m ℓ )

|  | Tc | SM | Ap | inodine method* |
|---|---|---|---|---|
| pMS504/CRK3000 | 100 | 6.25 | 0.025 | + + + |
| pMS506/CRK3000 | 100 | 100 | 0.025 | — |

| CRK | 1.6  6.25  0.025 | — |

---

* according to a method in " Protein Nucleic acid ,

Enzyme, " 23 (5) : ( 1978).

The above transformants were identifed by nitrocephine method which showing pMS504 producing a $\beta$-lactamase.

(B) Pseudomonas aeruginosa and Pseudomonas putida :

Plasmid pMS505-1 or pMS506 ( 25 $\mu$ $\ell$) obtained in the previous examples was mixed with competent cells ( 200 $\mu$ $\ell$) of Pseudomonas aeruginosa or Pseudomonas putida and reacted at 0 ℃ for 60 minutes, thereafter treated at 42℃ for 2 minutes. L-broth ( 1m $\ell$) was added thereto and incubated at 37 ℃ for 90 minutes. Cultured broth at 3,000 r.p.m. for 10 minutes, washed twice with BSG, further added BSG ( 0.5m $\ell$) thereto. This was selected by spreading on Lac-BTB medium containing streptomycin 12.5 $\mu$ g/m $\ell$ , further twice repeated the pure culture with medium containing streptomycin 25 $\mu$ g/m $\ell$ to obtain transformed pure culture strains of Pseudomonas aeruginosa and Pseudomonas putida .

MIC ( $\mu$ g/m $\ell$ ) of these strains ( $10^6$ cells/m $\ell$ ) is shown in Table 2 and 3.

Table 2  MIC on Pseudomonas aeruginosa

| | Tc | SM | Ap |
|---|---|---|---|

| pMS506/p · aerginosa | 50 | 100 | 12.5 |
|---|---|---|---|
| pMS505-1/p · aerginosa | 6.25 | >200 | >1600 |
| P. aeruginosa PAO 2142 | 6.25 | 1.6 | 12.5 |

Table 3  MIC on Pseudomonas putida

|  | Tc | SM | Ap |
|---|---|---|---|
| pMS506/p · putida | 50 | 200 | 100 |
| pMS505-1/p · putida | 1.6 | >200 | >1600 |
| P. putida Tn 1126 | 1.6 | 1.6 | 100 |

MIC of host cell E. coli used is shown in Table 4.

Table 4 MIC on E. coli

|  | Sm | Tc | Ap |
|---|---|---|---|
| pMS504/ML4901 | 0.4 | 6.25 | 800 |
| pMS506/ML4901 | 100 | 6.25 | 1.6 |
|  |  |  |  |

0218468

- 55 -

| pMS505-1/ML4901 | 100 | 0.8 | >1600 |
|---|---|---|---|
| E. coli ML4901 | 0.4 | 0.8 | 1.6 |

BRIEF EXPLANATION OF DRAWINGS :

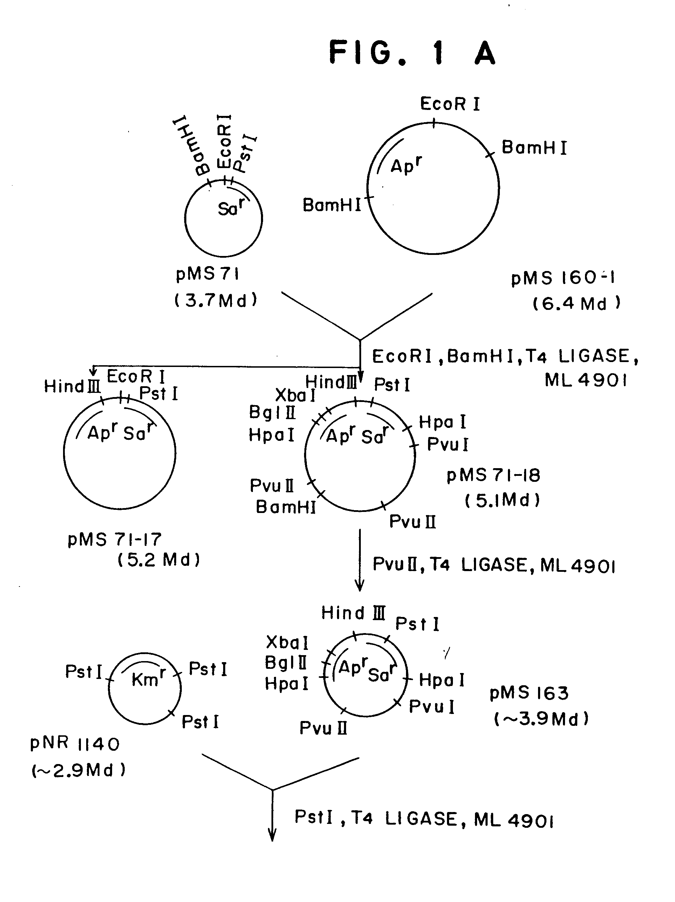

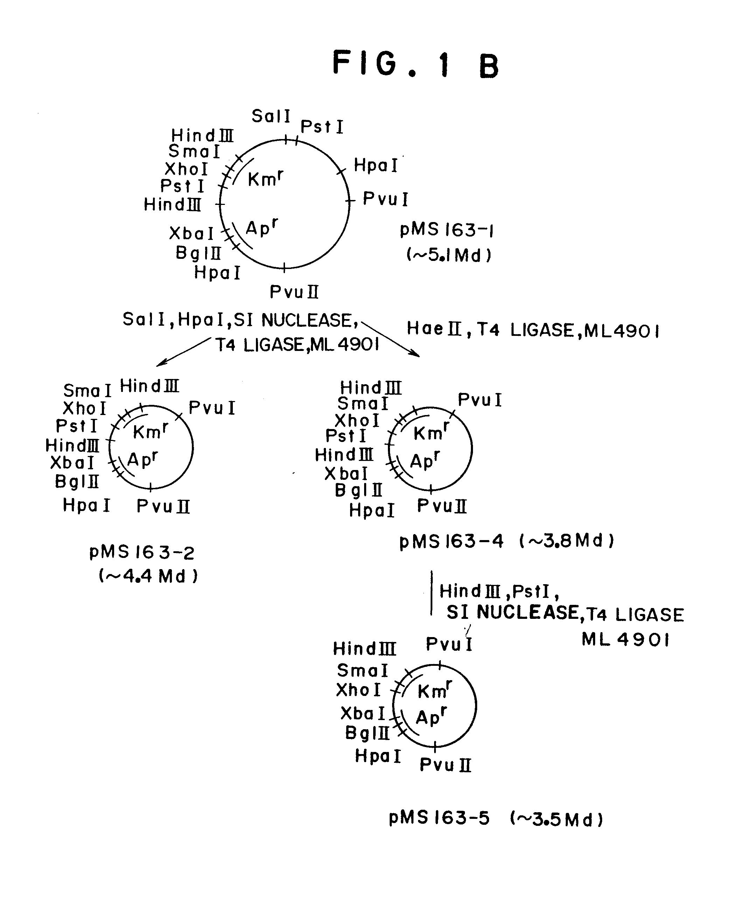

Fig. 1 (A)(B) : Restriction site of versatile vector Plasmid of the present invention functioned on E. coli and Pseudomonas, and process for preparation of plasmid.

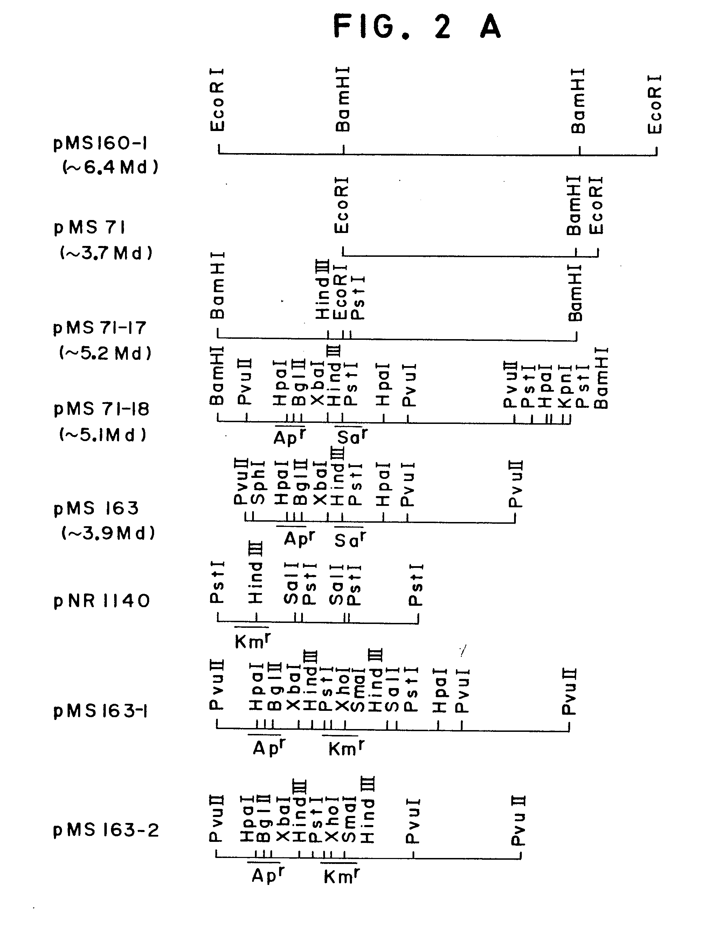

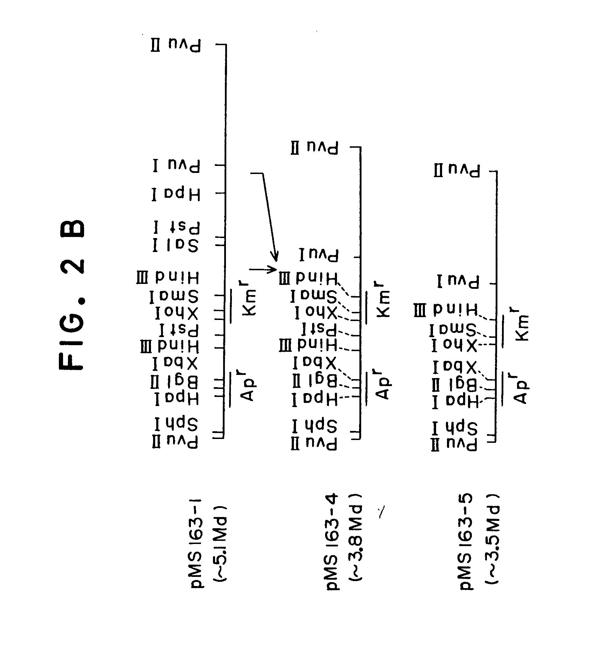

Fig. 2 (A)(B) : Restriction enzyme cleavage site and resistance region of plasmid in the process of preparing versatile vector of the present invention. 8 allows show that restriction enzyme cleavage site or each plasmid is transferred to next step plasmid )

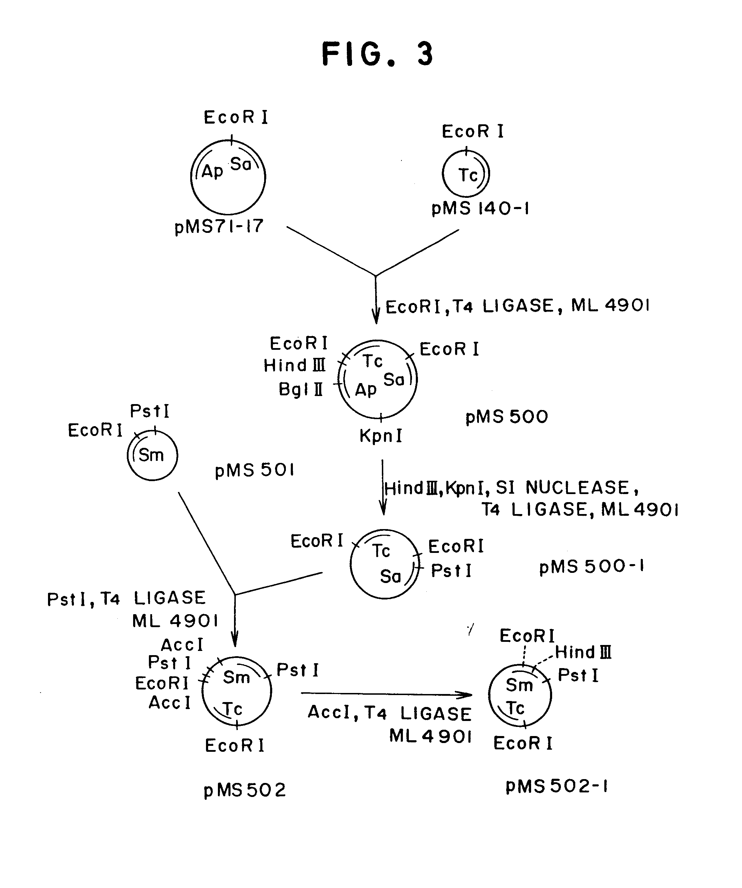

Fig. 3 : Restriction site of intermediate plasmids of the present invention and process for preparation of each plasmid.

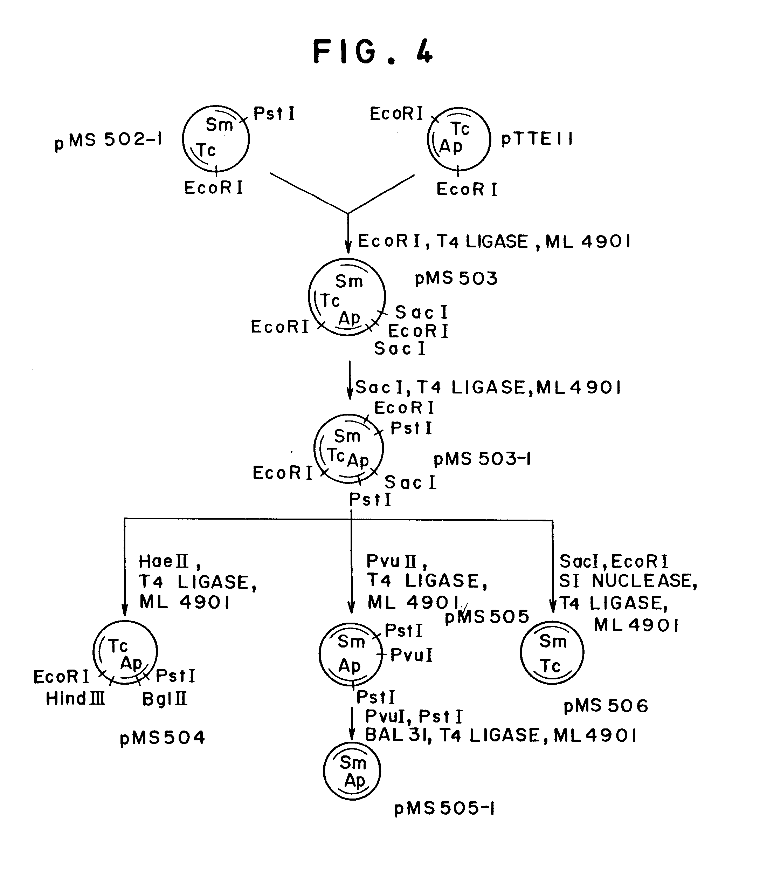

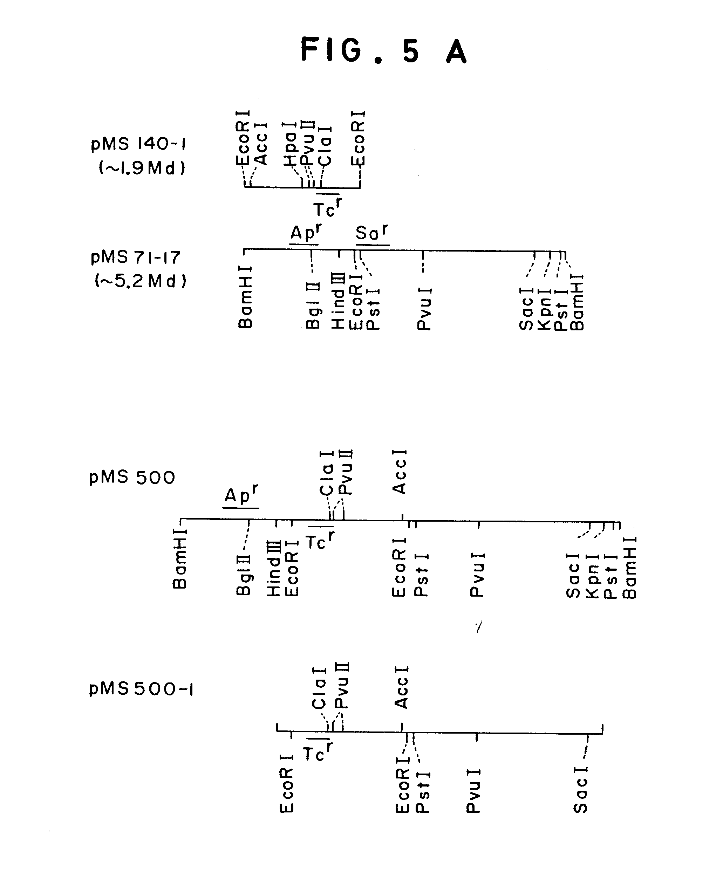

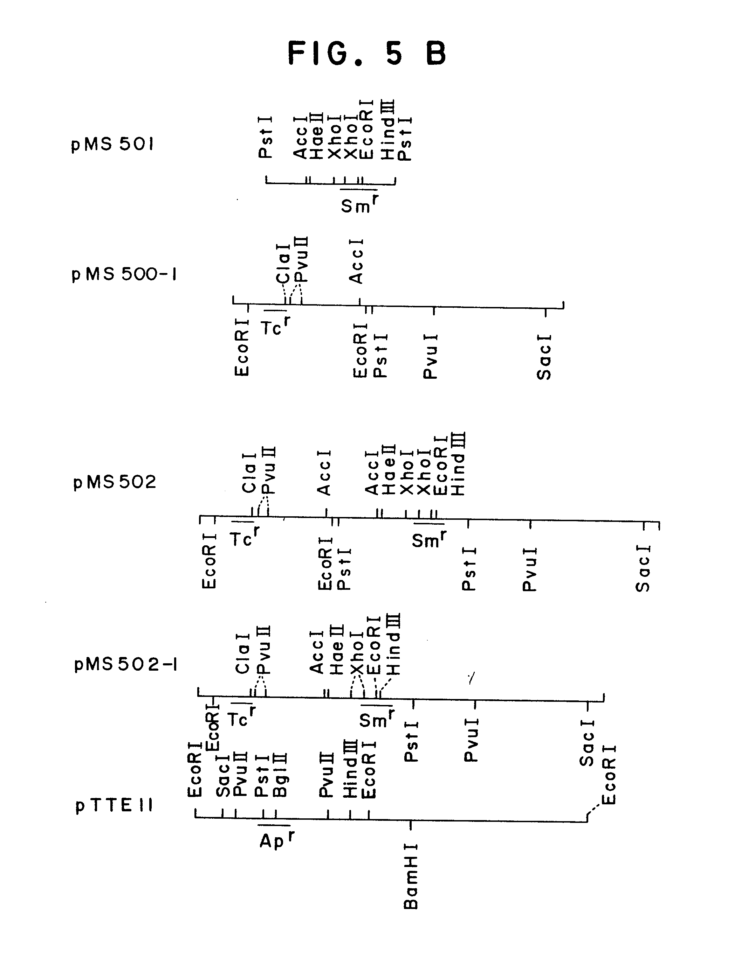

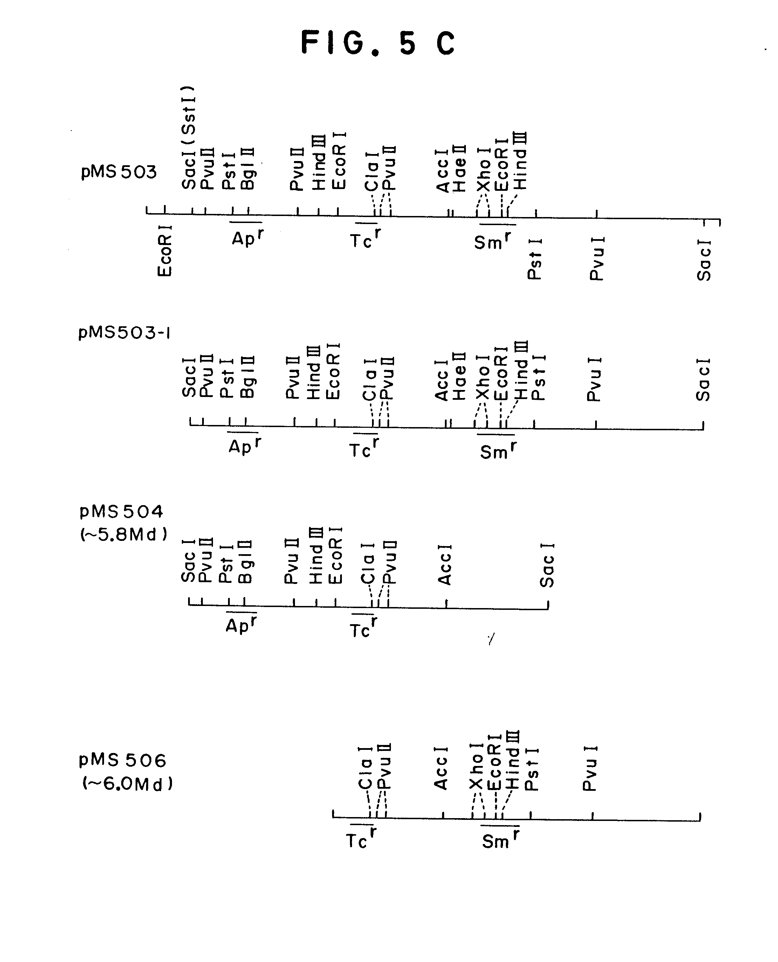

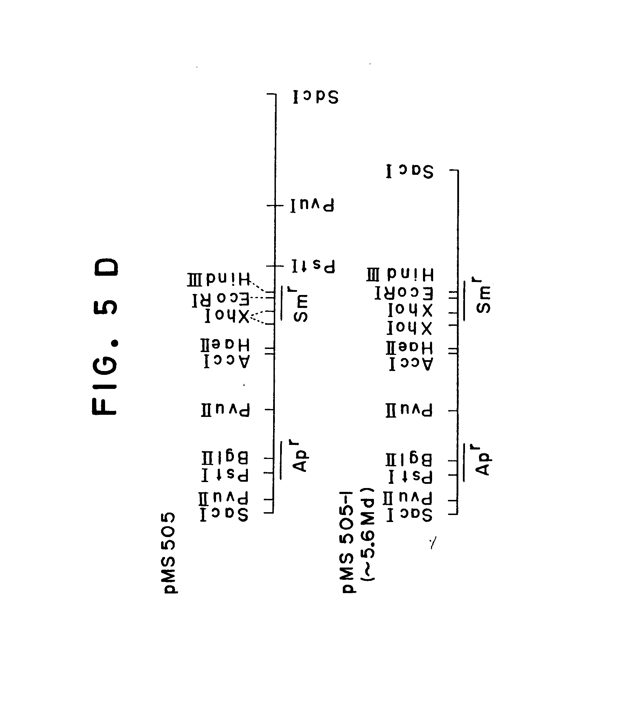

Fig. 4 : Restriction site of versatile plasmid vector of the present invention functioned in E. coli, Bcillus and Pseudomonas, and process for preparation of each plasmid.

Fig. 5 (A)(B)(C) :
Restriction enzyme sites and resistance regions of each plasmid in the process of preparing versatile vector plasmid of the present invention.

# 0218468

- 56 -

CLAIMS

1. A versatile recombinant DNA cloning vector having;

(a) two or more functionally different origins of replication selected independently from an origin of replication functionable in _Bacillus subtilis_, an origin of replication functionable in _Escherichia coli_ and an origin of replication functionable in _Pseudomonas aeruginosa_, and

(b) one or more DNA sequence which is capable of providing resistance to at least one antibiotic when a susceptible host cell in which a said origin of replication is capable of functioning is transformed by the vector.

2. A vector according to claim 1 wherein the origin of replication functionable in _Bacillus subtilis_ is a restriction fragment of a plasmid from a Gram positive bacteria and the origin of replication functionable in _Escherichia coli_ or _Pseudomonas aeruginosa_ is a restriction fragment of a plasmid from a Gram negative bacteria.

3. A vector according to claim 1 or 2 wherein the origins of replication are provided by restriction fragments of plasmids _pMS_ 140-1 and _pMS_ 71.

4. A vector according to claim 3 wherein a selectable marker gene chosen from an ampicillin resistance gene, streptomycin resistance gene and tetracycline resistance gene is provided.

5. A vector according to claim 4 wherein the selectable marker gene has originated from a chromosome or plasmid of Gram positive bacteria.

6. A vector according to claim 1 or 2 wherein an origin of replication is provided by a restriction fragment of _pMS_ 71.

7. A vector according to claim 6 wherein a selectable marker gene chosen from an ampicillin resistance gene, sulfa drug resistance gene and kanamycin resistance gene is provided.

8. A vector according to claim 1 which is pMS 504 ( A p$^r$ T c$^r$ ) or a derivative thereof.

9. A vector according to claim 1 which is pMS 505 ( A p$^r$ S m$^r$ ) or a derivative thereof.

10. A vector according to claim 1 which is pMS 163 ( A p$^r$ S a$^r$ ) or a derivative thereof.

11. A vector according to claim 1 which is pMS 163-2 ( A p$^r$ K m$^r$ ) or a derivative thereof.

12. A vector according to claim 1 which is pMS 163-5 ( A p$^r$ K m$^r$ ) or a derivative thereof.

13. A vector according to any one of the preceding claims which is provided with an ampicillin resistance gene encoding penicillinase type $\beta$ -lactamase.

14. A vector according to any one of claims 10 to 12 wherein the ampicillin resistance gene is provided with restriction sites for BglII, XbaI and HpaI.

15. A vector according to claim 1, which is pMS 506 ( T c$^r$ S m$^r$ ) or a derivative thereof.

16. A vector according to any one of claims 8,9, 14 and 15 wherein the said vector can exist (non-incompatibility) with pSC101, pCR1, pBR322, pACYC184 or pMB9 in an Escherichia coli host cell.

17. A vector according to any one of the preceding claims wherein the said vector is not mobilizable at coexisting with pMS76, analogous plasmid thereof and conjugatable plasmid.

18. A transformed non-restricting host cell containing a recombinant DNA cloning vector as claimed in any one of the preceding claims, the said host cell having been susceptible prior to transformation to the or each antibiotic to which resistance is provided by the vector and at least one of the origins of replication in the vector being functional in the said host cell.

0218468

- 58 -

19.  A transformed host cell according to claim 18, wherein the host cell is <u>Bacillus</u> <u>subtilis</u>, <u>Escherichia</u> <u>coli</u>, <u>Pseudomonas</u> <u>aeruginosa</u> or <u>Pseudomonas</u> <u>putida</u>.

FIG. 1 A

# FIG. 1 B

SalI, HpaI, SI NUCLEASE, T4 LIGASE, ML 4901

HaeII, T4 LIGASE, ML4901

pMS163-2 (~4.4 Md)

pMS163-4 (~3.8Md)

HindIII, PstI, SI NUCLEASE, T4 LIGASE ML 4901

pMS163-5 (~3.5Md)

# FIG. 2 A

0218468

4/10

# FIG. 2 B

**pMS 163-1 (~5.1 Md)** — Ap$^r$, Km$^r$

Pvu II · Sph I · Hpa I · Bgl II · Xba I · Hind III · Pst I · Xho I · Smal · Hind III · Sal I / Pst I · Hpa I · Pvu I · Pvu II

**pMS 163-4 (~3.8 Md)** — Ap$^r$, Km$^r$

Pvu II · Sph I · Hpa I · Bgl II · Xba I · Hind III · Pst I · Xho I · Smal · Hind III · Pvu I · Pvu II

**pMS 163-5 (~3.5 Md)** — Ap$^r$, Km$^r$

Pvu II · Sph I · Hpa I · Bgl II · Xba I · Smal · Xho I · Hind III · Pvu I · Pvu II

# FIG. 3

# FIG. 4

# FIG. 5 A

# FIG. 5 B

# FIG. 5 C

# FIG. 5 D

pMS 505

SacI
PvuII
PstI
BglII
PvuII
AccI
HaeII
XhoI
EcoRI
HindIII
PstI
PvuI
SdcI

Ap$^r$
Sm$^r$

pMS 505-I
(~5.6 Md )

SacI
PvuII
PstI
BglII
PvuII
AccI
HaeII
XhoI
XhoI
EcoRI
HindIII
SacI

Ap$^r$
Sm$^r$